Europäisches Patentamt

European Patent Office

Office européen des brevets

(19)

(11) Publication number: **0 153 044**
**B1**

(12) **EUROPEAN PATENT SPECIFICATION**

(45) Date of publication of patent specification: **05.10.88**

(51) Int. Cl.⁴: **A 61 F 5/44, B 65 D 33/16**

(21) Application number: **85300599.9**

(22) Date of filing: **29.01.85**

(54) Ostomy bag closure means.

(30) Priority: **01.02.84 US 576228**

(43) Date of publication of application:
**28.08.85 Bulletin 85/35**

(45) Publication of the grant of the patent:
**05.10.88 Bulletin 88/40**

(84) Designated Contracting States:
**AT BE CH DE FR GB IT LI LU NL SE**

(56) References cited:
**AT-B- 268 986**
**DE-A-2 515 159**
**DE-B-1 278 930**
**GB-A-1 133 304**

(73) Proprietor: **E.R. Squibb & Sons, Inc.**
**Lawrenceville-Princeton Road**
**Princeton, N.J. 08540 (US)**

(72) Inventor: **Ferguson, Keith T.**
**231 Katherine Street**
**Scotch Plains New Jersey (US)**

(74) Representative: **Cook, Anthony John et al**
**D. YOUNG & CO. 10, Staple Inn**
**London, WC1V 7RD (GB)**

Courier Press, Leamington Spa, England.

## Description

This invention relates to drainable ostomy bags and more particularly to a closure for effectively sealing the normally open end of such a bag.

Major abdominal surgery for a number of diseases involving different parts of the gastrointestinal and urinary tract can result in the patient being left with an abdominal stoma. For example, three types of abdominal stoma are the colostomy, the ileostomy, and the ileal conduit. In the case of an ileostomy, the ileal conduit and many colostomy operations, the patient is unable to control the passage of bodily waste material and must rely upon an appliance attached to their body to collect this material. Ileostomates and colostomates whose discharge is a liquid consistency employ systems in which the waste collecting pouch has a bottom opening that permits the contents to be emptied and the pouch reused. With such pouches, some type of a closure means that seals the bottom opening while the pouch is collecting discharge from the stoma is used.

It has been proposed in German Published Patent Application DE-A-2515159 that a bag such as a urine collecting bag can be made of two plastics films, and that these films can be connected together by co-operation of one member having a groove or furrow with a second member having a ridge or beading.

The present invention aims to provide an improved closure means for drainable collection bags such as ostomy bags.

According to the invention, there is provided a closure member for closing the open lower end of a drainable ostomy bag comprising channel means made from a deformable and resilient plastics material; mating means made from a deformable and resilient plastics material for mating and locking engagement with said channel means; characterised by a connecting member made from a deformable and resilient plastics material connected to said channel means and said mating means for holding said channel and said mating means in parallel and spaced apart relationship to one another when said closure member is open and for providing a hinge parallel to said channel means and said mating means for permitting closing of said closure member whereby said mating means can be brought into mating and locking engagement with said channel means with the whole width of said open lower end disposed therebetween.

In one preferred embodiment, the closure member is an integrally formed device such as an extrusion.

In a particular embodiment, the channel means comprises a pair of spaced apart walls extending away from the connecting member and forming the channel therebetween, and each wall includes a ledge which extends towards the opposite wall whereby the channel is narrowed at that point. A member defined by the mating means comprises a rod or tube having a substantially cylindrical cross-section which forces the walls apart as it passes by the ledges. The ledges serve to lock the cylindrical member within the channel once the cylindrical member is seated therein.

Brief description of the drawings

Fig. 1 is an elevational frontal view of an ostomy bag showing the opening for the stoma and coupling for use therewith.

Fig. 2 is a top planar view of a preferred embodiment closure member for use with drainable ostomy bags.

Fig. 3 is a side elevational view of the preferred embodiment closure member of Fig. 2.

Fig. 4 is an enlarged side elevational view of the preferred embodiment closure member of Figs. 2 and 3 shown in the closed position about the open lower end of a drainable ostomy bag.

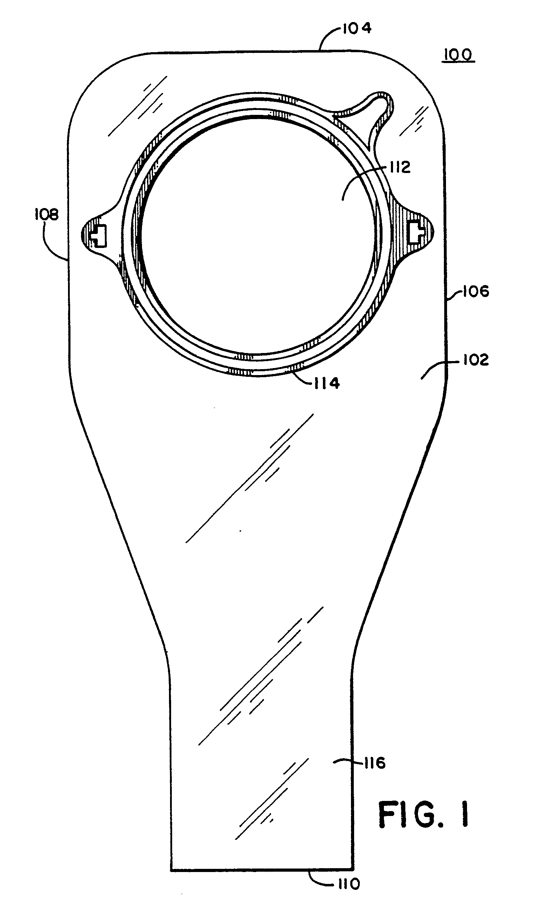

A typical ostomy bag 100 for which the preferred embodiment closure member of the present invention may be used is shown in Fig. 1 and is a bag-like article of flexible material, such as a clear, soft plastic or the like. It comprises front and back panels such as back panel 102 and may be formed by sealing the two panels together at the top 104 and at the sides 106 and 108. The bag is open at the bottom 110 so that discharge may pass through the open end of the bag for removal therefrom without the necessity for removing the bag from the body of a patient.

The back panel of the bag is typically provided with an opening 112 for positioning over the opening or stoma in the abdominal wall of a patient. The opening 112 is usually centrally located near the top of the bag.

Ostomy bags are usually secured to a pad or surgical dressing which contacts the patient's skin and surrounds the stoma. A coupling 114 is shown for use between the pad and bag which allows the bag to be readily removed when necessary and replaced by a clean, empty bag. The form of the coupling 114 is not essential to an understanding of the present invention and the particular coupling 114 shown in Fig. 1 is described in more detail in British Patent 1,571,657 entitled "Improvements in Ostomy Bag".

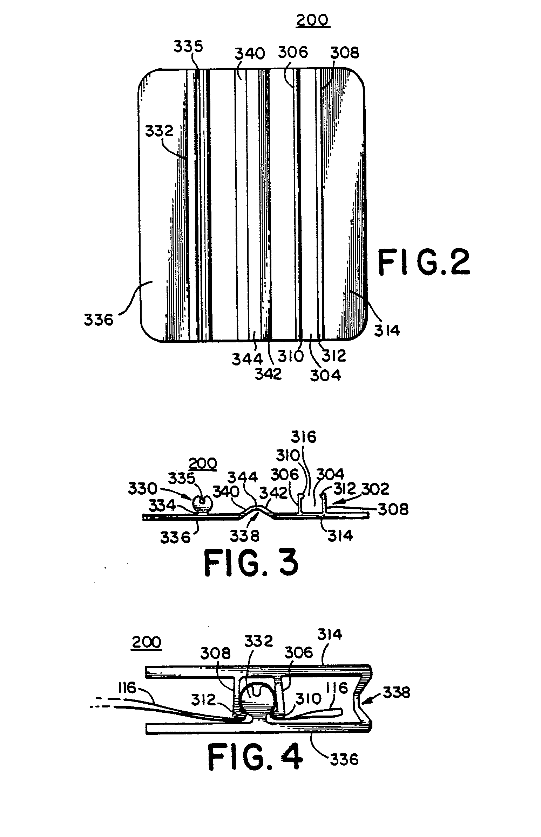

A preferred embodiment closure member designated generally 200 for use with drainable collection appliances such as the ostomy bag 100 of Fig. 1 is shown in Figs. 2 and 3. The closure member 200 comprises a channel means designated generally 302 made from a deformable but resilient synthetic plastic such as a high density polyethylene or polypropylene for defining a channel 304 and further including spaced apart walls 306 and 308 equipped with inwardly extending portions or ledges 310 and 312, respectively. The walls 306 and 308 are connected to a connecting member 314. Where the ledges 310 and 312 extend toward their respective opposite walls the channel 304 is narrowed in width at 316.

The closure member 200 further comprises a mating means designated generally 330 for defining a member 332 for mating and locking engagement with the channel 304. In the preferred embodiment, the member 332 is made of a deformable but resilient synthetic plastic such a high density polyethylene or polypropylene and comprises a slotted longitudinal rod connected to the connecting member 314 at 334. Even though a slightly elliptical cross-sectional slotted rod is shown in Fig. 3, a solid rod, a tube or a member of different cross-section such as circular or rectangular could be used with a correspondingly shaped channel.

The maximum cross-sectional dimension of the slotted rod member 332 is slightly larger than the narrowed channel width 316 at ledges 310 and 312 but slightly smaller than the width of the channel 304 below the ledges. The common connecting member 314 in the preferred embodiment is substantially planar having a substantial planar bottom surface 336 except in a hinge region intermediate the channel means 302 and mating means 330. There a longitudinal V-shaped portion 338 which is parallel to member 332 and channel 304 is formed by upwardly extending portions 340 and 342 which meet and form a raised portion 344. The V-shaped portion 338 allows the connecting member 314 to act more readily as a hinge when closure member 200 is closed by folding the channel means 302 and mating means 330 toward one another.

Fig. 4 is an enlarged side elevational view of the closure member 200 in the closed position about the tail or lower open end 116 of the drainable ostomy bag 100. To seal the bag the lower end 116 is positioned between the channel means 302 and the mating means 330 as the connecting member is folded. As the slotted rod member 332 passes by the ledges 310 and 312 it forces the walls 306 and 308 apart until the cylindrical tubular member 332 is fully seated in the channel 304 whereupon the resilient plastic walls return toward their original position when the closure member 200 was opened. At the same time the slot 335 in the member 332 allows the portion of the tubular member 332 on either side of the slot to bend inwardly when squeezing past the ledges 310 and 312 to assist in seating the member 332 in the channel 304. The ledges fit securely against the neck of the mating means 330 where the tubular member 332 is connected to connection member 330. The lower end 116 of the ostomy bag is clamped between the portions where the ledges 310 and 312 press against the neck of the mating means 330. A portion of the lower end 116 is shown below the wall 306 in the space above the hinge region 338 in Fig. 4. The hinge region 338 of the connecting member 314 acts as a hinge mentioned before for folding and unfolding the connecting member to close and open the closure member respectively. Other embodiments such as making the connecting member thinner in the region intermediate the mating means and channel means; providing a plurality of parallel and spaced apart grooves therein; or making the connecting member of a flexible enough material will serve to facilitate the hinging action required.

The channel means 302 and mating means 330 may be separate parts which are connected to a separate substantially planar connecting member 314. Such a connection may be made by an adhesive, for example. They can be glued or cemented to a rectangular plastic strip of high density polyethylene or polypropylene. However, the preferred embodiment of the closure member comprises integrally forming the channel means, mating means and connecting member as one piece in an extrusion process using high density polyethylene or polypropylene.

The connecting member serves to keep the channel 304 and tubular member 332 in a parallel and spaced apart relationship when the channel member is open so that as the connecting member 314 is folded the tubular member 332 easily aligns itself with the channel 304 for mating therewith. By integrally forming the closure member 200 in an extrusion like process, various length closure members suitable for various sized ostomy bags are readily and economically made.

The preferred embodiment connecting member of the present invention provides ease of use to ostomates for opening and closing ostomy bags for draining and resealing. Any such ease of use with such appliances is a welcome improvement.

There has been particularly disclosed herein an improved closure means for drainable bags which is easier to use and economical to manufacture. The preferred integral member closure device comprises an extrusion.

**Claims**

1. A closure member (200) for closing the open lower end (116) of a drainable ostomy bag comprising channel means (302) made from a deformable and resilient plastics material; mating means (330) made from a deformable and resilient plastics material for mating and locking engagement with said channel means; characterised by a connecting member (314) made from a deformable and resilient plastics material connected to said channel means (302) and said mating means (330) for holding said channel and said mating means in parallel and spaced apart relationship to one another when said closure member is open and for providing a hinge (344) parallel to said channel means and said mating means for permitting closing of said closure member (200) whereby said mating means can be brought into mating and locking engagement with said channel means with the whole width of said open lower end (116) disposed therebetween.

2. The closure member of claim 1 wherein said channel means, said mating means and said connecting member are integrally formed together.

3. The closure member of claim 2 wherein said closure member comprises an extrusion.

4. The closure member of claim 3 wherein said closure member comprises a high density polyethylene.

5. The closure member of claim 3 wherein said closure member comprises a high density polypropylene.

6. The closure member of claim 1 wherein said channel means comprises:

a pair of spaced apart walls (306, 308) extending away from said connecting member (314) and forming said channel (316) therebetween, each of said walls further comprising a ledge (310, 312) disposed on the top of said wall and extending toward the opposite wall whereby the width of said channel is reduced; and

wherein said member defined by said mating means further comprises a member (332) having a substantially cylindrical cross-section and in use said walls of said channel means are forced farther apart as said member is pressed by said ledges to allow said member to seat within said channel, whereafter said walls return to their original position and said ledges (310, 312) lock said member (332) in said channel (316).

7. The closure member of claim 6 wherein said member (316) has a slot (335) therein extending in a direction parallel to the length of the channel (316) to permit bending of the member (332) as it is squeezed past the ledges (310, 312).

## Patentansprüche

1. Verschluß (200) zum Verschließen des offenen unteren Endes (116) eines leerbaren Ostomiebeutels, der eine Kanaleinrichtung (302), die aus einem verformbaren und federnd nachgiebigen Kunststoffmaterial hergestellt ist, und eine Paßeinrichtung (330) aufweist, die aus einem verformbaren und federnd nachgiebigen Kunststoffmaterial zum Paß- und Festlegeeingriff mit der Kanaleinrichtung hergestellt ist, gekennzeichnet durch

ein Verbindungselement (314), das aus einem verformbaren und federnd nachgiebigen Kunststoffmaterial besteht, das mit der Kanaleinrichtung (302) und der Paßeinrichtung (330) derart verbunden ist, daß die Kanal- und die Paßeinrichtung parallel und im Abstand voneinander gehalten sind, wenn der Verschluß offen ist, und welches ein Gelenk (344) parallel zu der Kanaleinrichtung und der Paßeinrichtung bildet, um ein Schließen des Verschlußes (200) zu ermöglichen, wobei die Paßeinrichtung in Paß- und Festlegeeingriff mit der Kanaleinrichtung gebracht werden kann, bei dem die gesamte Breite des offenen unteren Endes (116) dazwischen angeordnet ist.

2. Verschluß nach Anspruch 1, bei dem die Kanaleinrichtung, die Paßeinrichtung und das Verbindungselement einteilig miteinander ausgebildet sind.

3. Verschluß nach Anspruch 2, bei dem der Verschluß ein Extrusionsteil aufweist.

4. Verschluß nach Anspruch 3, bei dem der Verschluß ein hochdichtes Polyethylen aufweist.

5. Verschluß nach Anspruch 3, bei dem der Verschluß ein hochdichtes Polypropylen aufweist.

6. Verschluß nach Anspruch 1, bei dem die Kanaleinrichtung aufweist:

ein Paar im Abstand angeordneter Wände (306, 308), die sich von dem Verbindungselement (314) wegerstrecken und dazwischen den Kanal (316) bilden, wobei jede Wand ferner einen vorspringenden Rand (310, 312) aufweist, der auf der Oberseite der Wand angeordnet ist und sich in Richtung zur gegenüberliegenden Wand erstreckt, wobei die Breite des Kanals vermindert wird, und

bei dem das von der Paßeinrichtung gebildete Teil ferner ein Teil (332) aufweist, das einen im wesentlichen zylindrischen Querschnitt hat, wobei im Gebrauchszustand die Wände der Kanaleinrichtung weiter voneinander weggedrückt werden, wenn das Teil durch den vorspringenden Rand gepreßt wird, um zu ermöglichen, daß das Teil in dem Kanal sitzt, und wobei anschließend die Wände in ihre Ausgangslage zurückkehren und die vorspringenden Ränder (310, 312) das Teil (332) in dem Kanal (316) festlegen.

7. Verschluß nach Anspruch 6, bei dem das Teil (316) einen Schlitz (335) hat, der darin in einer parallelen Richtung zur Länge des Kanals (316) verläuft, um ein Biegen des Teils (332) zu ermöglichen, wenn es an den vorspringenden Rändern (310, 312) vorbeigedrückt wird.

## Revendications

1. Elément de fermeture (200) destiné à fermer l'extrémité inférieure ouverte (116) d'une poche de stomie vidable, comprenant un canal (302) en matière plastique déformable et élastique; un moyen complémentaire (330) en matière plastique déformable et élastique, destiné à s'adapter et à se verrouiller sur ledit canal; caractérisé par

un élément de liaison (314) en matière plastique déformable et élastique, raccordé audit canal (302) et audit moyen complémentaire (330) pour maintenir ledit canal et ledit moyen complémentaire espacés parallèlement l'un de l'autre quand ledit élément de fermeture est ouvert, et pour former une charnière (344) paralléle audit canal et audit moyen complémentaire pour permettre la fermeture dudit élément de fermeture (200) de telle sorte que ledit moyen complémentaire peut être mis en prise d'adaptation et de verrouillage avec ledit canal, avec toute la largeur de ladite extrémité inférieure ouverte (116) disposée entre les deux.

2. Elément de fermeture selon la revendication 1, dans lequel ledit canal, ledit moyen complémentaire et ledit élément de liaison sont formés d'une seule pièce.

3. Elément de fermeture selon la revendication 2, dans lequel ledit élément de fermeture comprend une pièce extrudée.

4. Elément de fermeture selon la revendication 3, dans lequel ledit élément de fermeture est en polyéthylène haute densité.

5. Elément de fermeture selon la revendication 3, dans lequel ledit élément de fermeture est en polypropylène haute densité.

6. Elément de fermeture selon la revendication 1, dans lequel ledit canal comprend:

deux parois espacées (306, 308) partant dudit élément de liaison (314) et formant entre elles ledit canal (316), chacune desdites parois comprenant en outre un rebord (310, 312) disposé au sommet de ladite paroi et dirigé vers la paroi opposée de sorte que la largeur dudit canal est réduite; et

dans lequel ledit élément défini par ledit moyen complémentaire comprend en outre un élément (332) à section droite sensiblement cylindrique et, à l'utilisation, lesdites parois dudit canal sont forcées à s'écarter, à mesure que ledit élément est comprimé par lesdits rebords, pour permettre audit élément de se loger à l'intérieur dudit canal, après quoi lesdites parois reprennent leur position d'origine et lesdits rebords (310, 312) bloquent ledit élément (332) dans ledit canal (316).

7. Elément de fermeture selon la revendication 6, dans lequel ledit élément (316) est traversé d'une fente (335) parallèle à la longueur du canal (316) pour permettre à l'élément (332) de plier quand il est comprimé en passant entre les rebords (310, 312).

FIG. I

1

FIG.2

FIG. 3

FIG. 4